# EUROPÄISCHE PATENTSCHRIFT

(11) **EP 0 785 428 B1**
(45) Veröffentlichungstag und Bekanntmachung des Hinweises auf die Patenterteilung: **07.04.2004**
(21) Anmeldenummer: 97100865.1
(22) Anmeldetag: 21.01.1997
(51) Int. Cl.: G01N 33/487, C12M 1/34, B03C 5/02

(54) **Lagestabile Positionierung aktiv beweglicher Einzeller**
Stabilized lengthwise positioning of movablemonocellular bodies
Positionnement en longueur stabilisée de corps unicellulaires mobiles

(30) Priorität: 22.01.1996 DE 19602164; 16.02.1996 DE 19605830
(43) Veröffentlichungstag der Anmeldung: 23.07.1997
(73) Patentinhaber: Fraunhofer-Gesellschaft zur Förderung der angewandten Forschung e.V., 80686 München (DE); EPPENDORF-NETHELER-HINZ GMBH, D-22339 Hamburg (DE)
(72) Erfinder: Lucas, Kurt, 22761 Hamburg (DE); Fuhr, Günter, 13127 Berlin (DE); Müller, Torsten, 12439 Berlin (DE); Reimer, Klaus, 25524 Itzehoe (DE); Wagner, Bernd, 25582 Looft (DE)
(74) Vertreter: Leonhard, Frank Reimund, Dipl.-Ing.

(56) Entgegenhaltungen:
- WO-A-91/08284
- WO-A-92/07657
- DE-A- 4 400 955
- IEEE TRANSACTIONS ON INDUSTRY APPLICATIONS, Bd. 29, Nr. 2, 1.März 1993, Seiten 286-293, XP000369386 MASAO WASHIZU ET AL: "DIELECTROPHORETIC MEASUREMENT OF BACTERIAL MOTOR CHARACTERISTICS"

## Beschreibung

Das technische Gebiet der Erfindung sind planare und drei-dimensionale Mikroelektrodenanordnungen in Halbleiterchipgröße von einigen µm in einem Verfahren, das suspendierte, aktiv bewegliche (schwimmende) Einzeller, wie Spermien oder Bakterien, berührungslos in einer Suspension stabil positioniert. Ihre Schwimmund Stoffwechselaktivität kann dann bewertet und einzelne Organismen sortiert bzw. entnommen werden.

Zahlreiche biologisch/medizinische relevante Zelltechniken erfordern die Auswahl bestimmter Zellen aus einer sehr großen Menge von suspendierten Organismen. Eine Bewertung einzelner Zellen ist vor allem dann schwierig, wenn diese eine aktive Schwimmbewegung ausführen. Das trifft insbesondere für Bakterien und Spermien zu, die sich in elektrolytreichen Lösungen sehr schnell bewegen können (einige µm/s bis zu einigen mm/s). Die permanente, teilweise stochastische Ortsveränderung erschwert eine sichere Bewertung der Aktivität sich bewegender Mikroobjekte. Hinzu kommt, daß die Bewegung selbst häufig ein Maß für die Vitalität und Verwendbarkeit einzelner Zellen oder Mikroorganismen ist, z.B. bei der künstlichen Fertilisation.

Dem Stand der Technik entsprechen zwei Grundprinzipien, mit denen eine Einzelobjektcharakterisierung und Manipulation derzeit durchgeführt wird:
1. Die Immobilisierung, d.h. Retardierung oder komplette temporäre oder vollständige Bewegungshemmung erfolgt über die Applikation von biochemisch wirksamen Substanzen.
2. Die Verfolgung einzelner Objekte geht mittels technisch aufwendiger Videoauswertsysteme vonstatten, bei denen eine automatische Objektidentifikation erforderlich ist.
3. Die WO-A 92/07657 (Fraunhofer) veranschaulicht eine Vorrichtung und ein Verfahren mit einem Transportkanal, der sich verzweigen kann, vgl. dortige Figuren 7 und 6. Damit werden kleine dielektrische Teilchen gehandhabt, wobei die Teilchen einem elektrischen Feld ausgesetzt werden. Durch die Bahnführung werden die Teilchen separiert, an den Ort der Untersuchung bewegt, fokussiert und dort gehalten. Sie können berührungslos in einem elektrodenfreien Raum dazu gehalten werden, was man auch als eine "Teilchenweiche" bezeichnen kann.

Das erste Verfahren bedeutet einen nicht unerheblichen und insbesondere für medizinische Fragestellungen problematischen Eingriff in die Zellphysiologie. Genutzt werden üblicherweise pharmakologische Substanzen, aber auch Temperaturänderungen oder Viskositätserhöhungen. Das zweite Verfahren ist kostenaufwendig und entspricht einer dynamischen Bildauswertung mit sehr hohen Ansprüchen an die Objekterkennung.

Neben der Identifikation und Charakterisierung bestimmter Objekte spielt die gezielte Separation und Entnahme eine wichtige Rolle. Ein Beispiel **dieses Standes der Technik** ist die Entnahme eines Spermiums mit einer Mikropipette und die *in vitro* Befruchtung einer Eizelle nach Injektion des Spermiums in eine Oozyte (vgl. van Steirteghem, A. et al., Baillieres. Clin. Obstet. Gynaecol. 8, 1994, Seiten 85-93). Auch dieser Entnahme-Prozess wird durch die Eigenbewegung der Objekte stark erschwert oder ist nicht in einem akzeptablen Zeitraum (<1 Stunde) möglich. Hier hat sich die Immobilisierung der Objekte durchgesetzt, die jedoch aus Gründen der Zellbelastung bzw. Kontamination nicht befriedigen kann.

Der Erfindung **liegt die Aufgabe zugrunde,** aktiv bewegliche Einzeller in Mikrosystemen mit Hilfe hochfrequenter elektrischer Felder ohne Einschränkung ihrer natürlichen Beweglichkeit berührungslos zu positionieren (orts- oder lagestabil zu halten), um sie besser bewerten, separieren oder entnehmen zu können. Das wird mit dem Verfahren gemäß Anspruch 1 erreicht.

Das vorgeschlagene Verfahren kann bei der Diagnostik und Therapie von Befruchtungsproblemen, insbesondere zur künstlichen Befruchtung von Eizellen (in vitro Fertilisation) eingesetzt werden.

Mittels im Mikrometer- oder Submikrometerbereich in zwei typischen Dimensionen angeordnete Ausrichtungselektroden und Bremselektroden werden einzelne Einzeller - wie Spermien oder Bakterien - ausgerichtet und in ihrer Vorwärtsbewegung durch das Anlegen hochfrequenter elektrischer Felder so lange retardiert, bis eine gegenüber der Chipoberfläche ortsstabile Schwimmbewegung der Objekte erreicht ist. Vor einer oder mehreren Bremselektroden wird das Objekt gestoppt, ohne daß eine Berührung mit dem Substrat oder den Elektroden erfolgt. Die Positionierung kann durch eine der Schwimmrichtung der Objekte entgegengerichtete schwache Strömung unterstützt werden (Anspruch 2).

Ablenkelektroden (Anspruch 6) können ergänzend genutzt werden, um einzelne Objekte aus dem Beobachtungsraum in vorgebbarer Weise zu entfernen. Die Oberfläche zwischen und um die Elektroden herum kann im Mikrometerund Submikrometerbereich strukturiert sein (Mikrokanäle, Öffnungen, Vertiefungen etc.). Diese Strukturierung wird so ausgeführt, daß Mikrokapillaren oder andere Mikrowerkzeuge in das Mikrosystem eingeführt werden können, die geeignet sind, einzelne Objekte zu entnehmen und sie einer weiteren Verwendung zuzuführen.

Die Verwendung mikro-miniaturisierter Elektrodensysteme (typische Abmessungen im µm-Bereich) und die Applikation hochfrequenter elektrischer Felder grösser 100 kHz und mit einer Amplitude im mV bis V-Bereich ermöglicht die betastungsarme Positionierung der Objekte in physiologischen Lösungen, die in der Regel sehr hohe Leitfähigkeiten aufweisen (einige S/m). Das ist möglich durch eine geringere Wärmeproduktion und eine bessere Wärmeableitung in Halbleitersubstraten.

Die berührungslose Positionierung erlaubt es, die Einzeller zu charakterisieren (auch hinsichtlich der Bewegung der aktiven Organellen, wie Geiseln, Flagellen und Cilien) und sie frei schwebend in einer physiologischen Lösung selektiv zu entnehmen.

Die abhängigen Ansprüche konkretisieren und erweitern den abstrakten - von Anspruch 1 in seinen tragenden Merkmalen umrissenen - Gedanken der elektrisch steuerbaren, berührungslosen Positionierung einzelner sich aktiv bewegender Mikroorganismen oder Einzeller in dreidimensional angeordneten und mit leitfähigen Elektroden mikrostrukturierten Substraten.

Erfindungsgemäß werden die Ausrichtungs-Mikroelektroden so angeordnet, daß die Objekte gezwungen werden, in eine festgelegte Richtung zu schwimmen. In dieser Richtung nehmen die Feldkräfte linear, aperiodisch oder diskontinuierlich zu. Am Ende dieser Ausrichtungsstrecke wird eine so starke Gegenkraft erzeugt, daß die Objekte trotz weiterer aktiver Bewegung nicht vorwärts kommen. Das elektrische Feld wird dabei so gewählt (im MHz-Bereich), daß die Zellsysteme nicht oder nur geringfügig physiologisch beeinflußt sind, d.h. das Feld nicht wahrnehmen.

Da die Feldkräfte bei schnell schwimmenden Objekten, wie z.B. Spermien, nicht ganz ausreichen, die Nettobewegung gegenüber einer still stehenden Suspension vollständig zu kompensieren, kann eine entgegengerichtete Lösungströmung unterstützend genutzt werden (Anspruch 2). Diese Strömung wird entweder über die elektrische Ansteuerung, die Geometrie und die Anordnung der Mikroelektroden selbst erzeugt, oder von außen auf bekannte Weise (Pumpen) induziert. Vorrichtungen und Verfahren zu elektrisch über streifenförmige Elektroden aufgebrachten Strömungen sind in **J. Micromech. Microeng. 4** (1994), Seiten 217-226 beschrieben. Auch die WO-A 92/07657 beschreibt Möglichkeiten der Handhabung dielektrischer Teilchen.

Auf diese Weise entsteht eine elektrisch-hydrodynamische Positionierung (Anspruch 12), die durch Chemotaxis oder Phototaxis ergänzbar ist.

Durch Abschalten der Bremselektrode kann das schwimmende Objekt den Elektrodenbereich verlassen. Um auch hier die Richtung beeinflussen zu können, können weitere Mikroelektroden (Ablenkelektroden) vorgesehen sein, sind jedoch nicht zwingend erforderlich (Anspruch 6). Mittels dieser Elektroden oder in Kombination mit den anderen Elektroden läßt sich über ein ebenfalls hochfrequentes Feld oder kurze elektrische Impulse das Objekt in eine Richtung bewegen, die durch die Elektrodenansteuerung festgelegt und gesteuert wird z.B. durch Microprozessor.

Das ganze System kann als planar strukturierte Substratoberfläche (Glas, Silizium, Keramik, Kunststoff) oder als geschlossenes Mikrokanalsystem mit Zu- und Abflüssen, sowie Öffnungen zum Einführen einer Entnahmevorrichtung ausgeführt werden. Die Substratstrukturierungen und Prozessierungen der Elektroden können mit Techniken der Halbleiterherstellung erzeugt werden. Sie sind zumeist flach mittels mikromechanischer Fügetechniken (z.B. Kleben oder anodisches Bonden) zu Hybridsystemen zusammengefügt. Die Retardierstrecke wird günstig langgestreckt (verglichen mit der Kanalbreite) ausgebildet und endet stirnseitig an der den Kanal beendenden Wand, in deren Nähe die Bremeselektrode(n) plaziert sind.

Eine Beobachtung (z.B. mikroskopisch) ist von einer oder zwei Seiten möglich und sinnvoll. Bestimmte Bereiche der Siliziumchips können dabei auch so dünn ausgeführt werden (einige µm), daß sie optisch transparent werden.

Die Mikrokanalsysteme können Verzweigungen, Kreuzungen und Einmündungen aufweisen, über die die Objekte eingespült oder entnommen werden. Die Objektfangund Positionier-Elemente können nebeneinander, hintereinander oder kaskadenförmig mit Rückführungen mehrfach ausgeführt werden, um die Effizienz des Verfahrens zu verbessern.

Für spezielle Aufgaben können die Elektroden und Kanalabmessungen auch so gewählt werden, daß zwei oder mehr der schwimmenden Objekte nebeneinander gehalten werden können oder zu Gruppen vereinigt werden (Anspruch 3).

Zur Vermeidung von Feldverlusten und zur Reduktion der Wärmeerzeugung können Teile der Elektroden mit isolierenden Schichten überzogen werden.

Bevorzugte Ausführungsbeispiele der Erfindung werden nachfolgend unter Bezugnahme auf die beiliegenden Figuren näher erläutert.
- **Figur 1**: ist eine ebene Gestaltung von Elektroden 11a,11b auf einem Substrat zur ortsstabilen Fixierung eines Einzellers 15.
- **Figur 2**: ist eine mehrdimensionale Gestaltung von Elektroden 21 a,21 b,21 c,21 d.
- **Figur 3**: ist eine Parallelschaltung der Brems- und Halteeinrichtung gemäß Figur 1 oder Figur 2.
- **Figur 4**: ist eine Ausführung von Figur 1 ohne mechanische Nut 16, nur begrenzt durch jeweilige Ausrichtungselektroden 41a,41b.

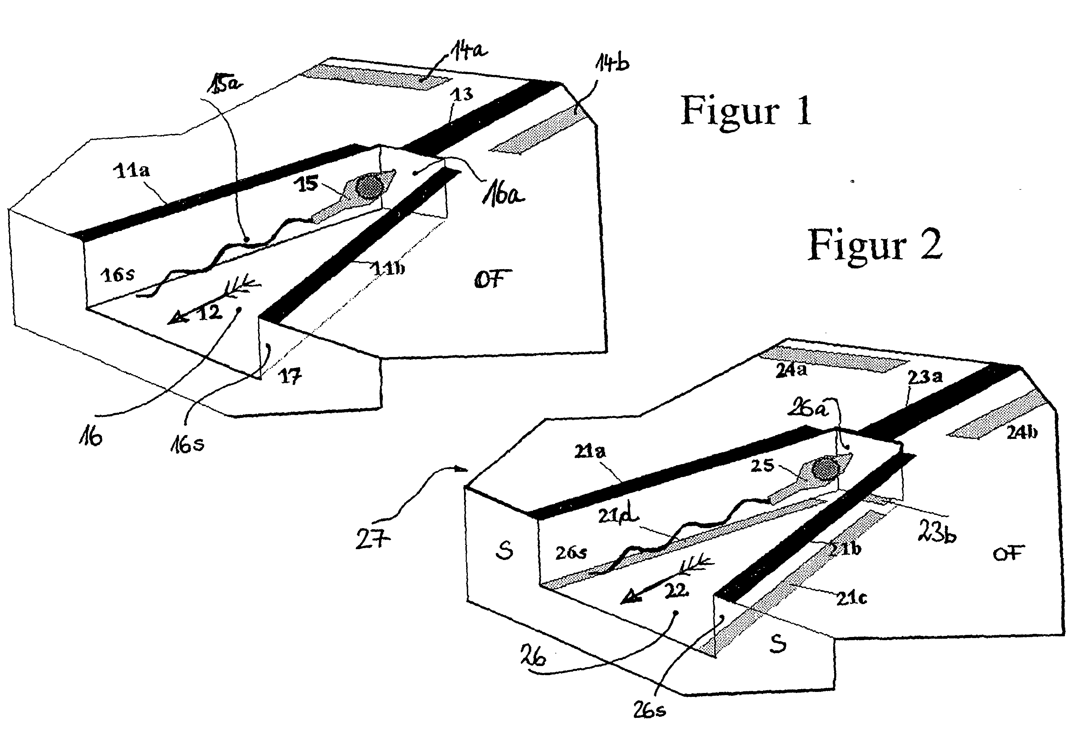

Figur 1 veranschaulicht einen Auschnitt aus einer mikrostrukturierten Oberfläche eines Substrates 17. In das Substrat ist eine als Kanal 16 mit verschlossenem Ende (Wand) 16a ausgebildete Nut eingebracht (z.B. geätzt), deren Seitenwände 16s zum verschlossenen Ende 16a hin verlaufen. Das stumpfe Ende des Kanales ist im Querschnitt am Geringsten, mit Blick auf alle anderen Kanalquerschnitte. In besonderer Gestaltung kann der Kanal 16 mit flachen Seitenwänden 16s konisch auf das stumpfe Ende 16a zulaufen.

In dem Kanal 16 wird eine Flüssigkeitsströmung 12 erzeugt. Die Flüssigkeitsströmung kann über streifenförmig angeordnete Elektroden erzeugt werden, mit denen hochfrequente Wanderfelder gebildet werden (vgl. die eingangs genannte Veröffentlichung aus Journal of Micromechanics and Microengineering, Heft 4, 1994, Seiten 217ff). Ab einer bestimmten Amplitude tritt eine lokale Erwärmung der Flüssigkeit über den streifenförmigen Elektroden auf, welche Elektroden in der Fig. 1 nicht dargestellt sind, aber gedanklich leicht ergänzt werden können. Die lokale Erwärmung führt zu einem Leitfähigkeitsgradienten oberhalb der Elektroden, beispielsweise im dargestellten Kanal 16, an dessen Boden die streifenförmigen Elektroden angebracht sind. In den nun anisotropen Flüssigkeiten lassen sich über elektrische Felder - die zusätzlich induziert werden - Raumladungen erzeugen, welche in dem erwähnten elektrischen Wanderfeld bewegt werden, um die Flüssigkeitsströmung im Kanal 16 zu erzeugen. Der erwähnte Leitfähigkeitsgradient kann auch über einen anders induzierten Temperaturgradienten gebildet werden, namentlich auf nicht-elektrischem Wege, z.B. durch Kühlen auf der Unterseite des Kanals 16 und Heizen im Abstand davon auf der Oberseite.

Mit beiden Varianten lassen sich Strömungen zwischen 50 µm/sec bis hin zu 300 µm/sec erzeugen, die den Geschwindigkeiten entsprechen, mit denen man ergänzende Bremskräfte auf die Spermien 15 aufbringen möchte.

Entgegen der Strömung wird ein Spermium 15 mittels eines hochfrequenten elektrischen Feldes, das zwischen Ausrichtungselektroden 11a und 11 b und einer Bremselektrode 13 erzeugt wird, ausgerichtet und durch die konische Anordnung der Elektroden 11 a, 11b zu der Bremselektrode 13 gesteuert geführt.

In der gezeigten Position des Spermiums 15 wäre die über die Ausrichtungs- und Bremselektrode 13 erzeugte dielektrophoretische Gegenkraft so groß, daß die Zelle trotz aktiver Bewegung der Geisel 15a keine Nettobewegung gegenüber der Chip-Oberfläche OF ausführen kann. Das Objekt 15 schwimmt dann ortstabil ohne Beeinträchtigung seiner Beweglichkeit. Befindet sich ober- oder unterhalb des Kanal-Bereiches 16 ein Detektor (z.B. optisch,elektrisch) kann die Schwimmbewegung quantitativ erfaßt werden. Sobald die Bremselektrode 13 abgeschaltet wird, verläßt das Spermium den Bereich in eine Richtung, entgegengesetzt zur Strömung 12.

Die elektrische Ansteuerung der Elektroden 11 a,11 b und der Bremselektrode 13 in der Fig. 1 werden später im Zusammenhang mit den entsprechenden Elektroden 21 a-21d und 23a,23b der Fig. 2 erläutert.

Über weitere Ablenkelektroden 14a und 14b, kann das Objekt 15 über die Chipoberfläche OF gelenkt werden.

Die dargestellte Anordnung kann in verschiedenen Größenausführungen und Anordnungsmodifikationen auf spezielle Zellen adaptiert werden.

Das Substrat 17 kann Glas, ein Halbleitermaterial, Kunststoff oder Keramik sein. Auch andere glatte, strukturierbare Oberflächen sind denkbar.

Die ortsstabile berührungslose Fixierung funktioniert auch, wenn keine Gegenströmung 12 erzeugt wird, allein über eine Applikation hochfrequenter elektrischer Felder (Frequenzen zwischen 100 kHz und 1 GHz und Amplituden von einigen 100 mV bis zu einigen Volt). Mit den hohen Frequenzen werden die induzierten Membranpotentiale gering gehalten. Sie liegen bei den zur Lagefixierung notwendigen Feldstärken im Bereich von einigen mV. Die planare Ausführung der Elektroden 11 a,11 b,13 verbessert die Wärmeableitung in die Flüssigkeit und in das Substrat, bei gleichzeitiger Steigerung der Feldgradienten.

Teile der Chipoberfläche 17 können mit einem weiteren Substrat (strukturiert oder unstrukturiert) bedeckt werden, so daß geschlossene Mikrosysteme entstehen. Im einfachsten Fall handelt es sich um ein Glasplättchen. In Frage kommen beispielsweise photolithographische oder elektronenstrahl-lithographische Prozessierungen und eine Verbindung über anodisches Bonden.

**Figur 2** zeigt einen zu Figur 1 ähnlichen Aufbau, mit dem Unterschied, daß hier eine 3-dimensionale Struktur vorliegt, indem zwei Elektrodenebenen (erste Ebene: 21a,21 b,23a,24a,24b; zweite Ebene: 21 c,21d,23b) prozessiert wurden, die über strukturierte Spacer S zur Bildung eines Kanals 26 beabstandet und isoliert sind. Diese Spacer S erzeugen gleichzeitig ein Microkanalsystem 26. Wieder kann mit einer Gegenströmung 22 gearbeitet werden. Das Stirnende des Kanals 26 ist 26a, an dem die Spacer S enden. Die freien Seiten der Spacer zum Kanal 26 bilden seine Seitenwände 26s.

Das Spermium 25 wird in einer Quadrupol-Feldfalle 21a,21b,21c und 21 d gehalten und vor zwei beabstandeten Bremselektroden 23a und 23b positioniert. Auch hier können weitere Ablenkelektroden 24a und 24b verwendet werden. Der Chip kann offen oder mit einer Platte (Glas, Halbleiter usw.) bedeckt und verschlossen werden. Über eingefügte Öffnungen oder in den Kanal 26 kann eine Mikrokapillare eingeführt werden. Diese würde das Spermium mit der Geisel zuerst aufnehmen und seine Entnahme aus der Halterungsposition ermöglichen.

Die elektrische Ansteuerung der Führungselektroden 21, die konisch zulaufen, und der Bremselektroden 23 in den **Figuren 1 und 2** soll gemeinsam beschrieben werden. Die Elektroden am Kanal 16 bzw. 26, die die Ausrichtung und gesteuerte Zuführung der Spermien 15 zur Bremselektrode bewirken, werden mit Wechselspannung betrieben. In **Fig. 1** liegen dazu die Ausrichtungselektroden 11a, 11b an derselben Phase an und die Bremselektrode 13 an der anderen Phase, wobei die beiden Phasen um 180° gegeneinander verschoben sind. Als Spannungsform eignen sich Rechteckspannung, Sinusspannung oder Dreiecksspannung, auch andere Formen, insbesondere solche, die sich aus den vorgenannten zusammensetzen, können verwendet werden.

Es sollten zur zuverlässigen Funktionsweise periodische Signale verwendet werden.

In gleicher Weise kann die Anordnung gemäß Fig. 2 betrieben werden, wenn an die Elektroden 21a und 21b (in der ersten Ebene) und die Bremselektrode 23b (in der zweiten Ebene) die Spannung mit der ersten Phasenlage angelegt wird und an die Elektroden 21d und 21c (in der zweiten Ebene) und die Bremselektrode 23a (in der ersten Ebene) die Spannung mit der um 180° verschobenen Phasenlage angelegt wird. Dann ergibt sich das Wechselfeld der Fig.1, nur stärker.

Die Fig. 2 eröffnet auch die Betriebsweise, ein Rotationsfeld anzulegen, wobei die vier Elektroden 21a-21d mit individuellen Spannungen betrieben werden, die jeweils um 90° verschoben sind. Werden mehr als vier Elektroden verwendet, so ergibt sich die notwendige Phasenlage aus 360° geteilt durch die Anzahl der Elektroden. Die Bremselektrode wird jeweils an diejenige Phase angelegt, die einer der Ausrichtungselektroden derselben Ebene entspricht, also z.B. an die Spannung gemäß der Ausrichtungselektrode 21 a hinsichtlich der Bremselektrode 23a für den Fall der vier Ausrichtungselektroden von Fig. 2. Die in der anderen Ebene gelegene Bremselektrode 23b würde an die Spannung angelegt werden, die auch die Elektrode 21c speist.

Ohne große Einbußen des wirksamen Rotationsfeldes kann auch die jeweils in der Ebene benachbarte Elektrode (21 b oder 21 d) verwendet werden.

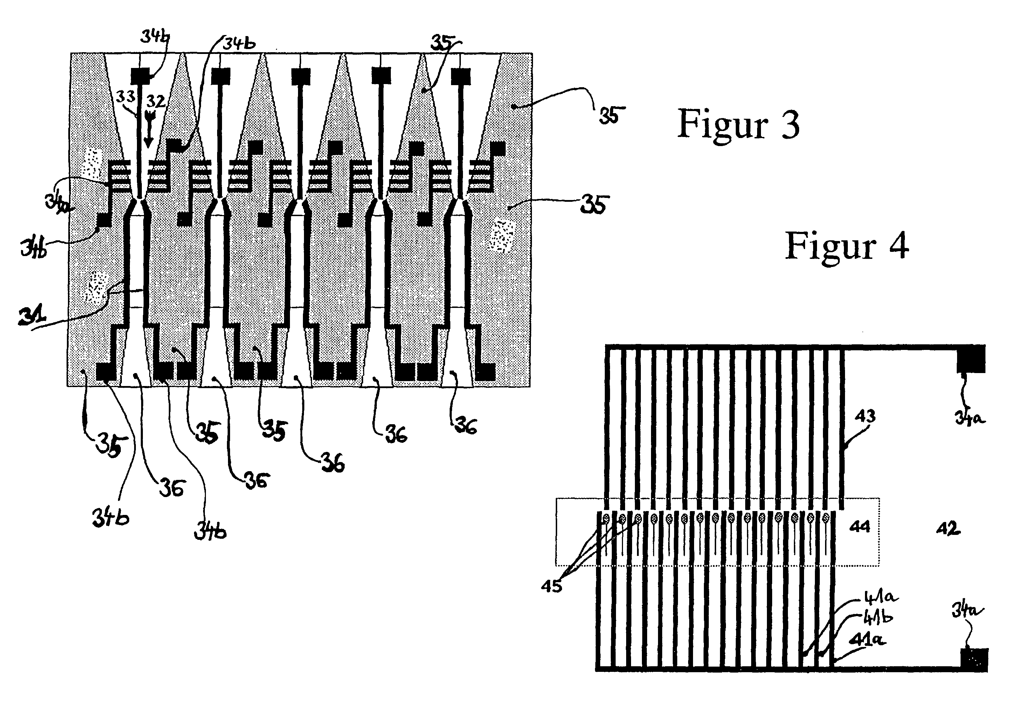

**Figur 3** ist ein Beispiel einer Mehrfachausführung der in Figur 1 und Figur 2 gezeigten Vorrichtung. Die Ausrichtungselektroden 31, die Bremselektroden 33 und die Ablenkelektroden 34 sind planar mit den Methoden der Halbleitertechnologie prozessiert. Der gepunktet dargestellte Bereich 35 kann ein Spacer, ein Isolator oder ein anderes Material sein, das die Mikrokanäle 36 begrenzt. Die schwarzen Vierecke 34b an den Elektroden 34a sind Bondflächen. Die Mikroorganismen oder Einzeller werden von unten in das Kanalsystem schwimmend gelenkt und geführt von den konvergierenden Elektroden 31, schließlich gehalten von dem vorderen Endabschnitt der konvergierenden Elektroden 31 und dem benachbarten Frontabschnitt der Bremselektrode 33. Die Gegenströmung 32 kann ergänzend hinzutreten.

Nach oben werden die Einzeller aus der Positionierung entlassen, gesteuert von den oberen Elektroden 34a mit Bondflächen 34b.

Der elektrische Anschluß über die Bondflächen 34a an allen Elektroden 31,34a,33 erfolgt wie in elektronischen Schaltkreisen. Entsprechende Systeme sind sterilisierbar.

**Figur 4** ist ein Design einer Positioniereinrichtung für 15 Einzelobjekte 45. Das Substrat ist mit 42 bezeichnet. Die darauf angebrachten Ausrichtungselektroden 41 bilden gleichzeitig die Einzelkanalbegrenzung (z.B. Prozessierung der Elektroden mit einer Dicke von einigen Mikrometern). Die jeweils zwischen den vorderen Enden der Ausrichtungselektroden endenden Bremselektroden 43 führen zu der Aufreihung der Zellen 45 in der gezeigten Weise. Unterhalb oder oberhalb des Zentralbereiches in dem die Enden der Elektroden eng benachbart sind, befindet sich ein Detektor 44, mit dem die Schwimmbewegung einzelner oder aller Objekte 45 erfaßt werden kann. Auch hier kann wieder ein partieller oder vollständiger Abschluß/Verschluß der Oberfläche vorgenommen werden.

## Patentansprüche

1. **Verfahren** zur stabilen Positionierung oder zur Separation aktiv beweglicher Einzeller, in flüssigen Medien, wobei die Einzeller (15,25,45)
(a) über eine oder mehrere im Mikrometerbereich langgestreckte Elektroden (11,21,31,41) mittels wechselnder oder wandernder hochfrequenter Felder ausgerichtet und abgebremst werden;
(b) mit einer oder mehreren - in ihren typischen Abmessungen den Abmessungen der Ausrichtungs-Elektroden (11,21,31,41) entsprechenden - Bremselektroden (13,23,33,43) in ihrer Vorwärtsbewegung retardiert bis ganz aufgehalten werden, um in diesem Positionier-Zustand Organellenbewegung(en) registrieren und bewerten zu können oder selektiv einzelne Einzeller mittels einzubringender Mikroelemente entnehmen zu können.

2. Verfahren nach Anspruch 1, **dadurch gekennzeichnet, daß** eine den Einzellern (15,25,45) entgegengerichtete Strömung (12,22,32) von außen oder über eine elektrische Ansteuerung der Mikroelektroden (11,21,31,41) erzeugt wird.

3. Verfahren nach einem der vorigen Verfahrensansprüche, **dadurch gekennzeichnet, daß** zwei oder mehr Einzeller gleichzeitig positioniert oder zu Gruppen formiert werden.

4. Verfahren nach einem der vorigen Verfahrensansprüche, bei dem die einzelnen Einzeller selektiv mittels Kapillaren entnommen werden.

5. Verfahren nach einem der vorigen Verfahrensansprüche bei dem die Organellenbewegung die Bewegung von Geiseln, Cilien oder Flagellen (15a) ist.

6. Verfahren nach einem der vorigen Verfahrensansprüche, **dadurch gekennzeichnet, daß** über miniaturisierte Ablenkelektroden (14,25,34) die Einzeller (15,24,45) nach ihrer Positionierung separiert oder aus ihrer Schwimmrichtung nach Entlassen aus der stabil gehaltenen Lage abgelenkt werden.

7. Verfahren nach einem der vorigen Verfahrensansprüche, **dadurch gekennzeichnet, daß** in dem Bereich, in dem die antreibende Kraft der Einzellerbewegung gerade kompensiert wird, Detektoren (44) oder optische Fenster vorgesehen werden, mit denen die die Einzeller charakterisierenden Parameter, insbesondere die Quantifizierung ihrer Schwimmbewegung, erfaßt werden.

8. Verfahren nach einem der vorigen Verfahrensansprüche, **dadurch gekennzeichnet, daß** einige der bewerieten Einzeller separiert und in ein abgetrenntes Mikrokompartiment überführt werden.

9. Verfahren nach einem der vorigen Verfahrensansprüche, **dadurch gekennzeichnet, daß** die Bremselektroden (13,23,33,43) und Ablenkelektroden (14,24,34) über einen Computer oder Mikroprozessor angesteuert oder gesteuert werden.

10. Verfahren nach einem der vorigen Verfahrensansprüche, **dadurch gekennzeichnet, daß** die elektrischen Wechselfelder oder wandernden Felder an den Elektroden (11a, 11b; 13) über Signale einer Frequenz groesser 0.1 MHz mit Amplituden zwischen 0,1 V und 40V erzeugt werden.

11. Verfahren nach einem der vorigen Verfahrensansprüche, **dadurch gekennzeichnet, daß** als bewegliche Einzeller Bakterien oder Spermien der verschiedensten Tierarten oder menschliche Spermien eingesetzt werden.

12. Verfahren nach einem der vorigen Verfahrensansprüche, **dadurch gekennzeichnet, daß** zusätzlich zu der elektrisch-hydrodynamischen Halterung der beweglichen Einzeller chemische oder optische Signale ausgesendet werden, um die Einzeller zu einer Richtungsänderung zu veranlassen.

## Claims

1. Process for stable positioning or for separation of actively moveable monocellular organisms in liquid media, wherein the monocellular organisms (15,25,45)
(a) are aligned and braked via one or more elongated electrodes (11,21, 31, 41) in the micrometre range by means of alternating or migrating high-frequency fields;
(b) are retarded to completely stopped in their forward movement by one or more braking electrodes (13, 23, 33, 43) - corresponding in their typical dimensions to the dimensions of the alignment electrodes (11, 21, 31, 41), in order to be able to register and evaluate organelle movement(s) in this positioning state or to be able to selectively remove individual monocellular organisms by means of microelements to be introduced.

2. Process according to claim 1, **characterised in that** a stream (12, 22, 32) directed counter to the monocellular organisms (15, 25, 45) is produced externally or via electrical control of the microelectrodes (11, 21, 31, 41).

3. Process according to one of the previous process claims, **characterised in that** two or more monocellular organisms are positioned at the same time or formed into groups.

4. Process according to one of the previous process claims, in which the individual monocellular organisms are removed selectively by means of capillaries.

5. Process according to one of the previous process claims, in which the organelle movement is the movement of whips, cilia or flagella (15a).

6. Process according to one of the previous process claims, **characterised in that** the monocellular organisms (15, 24, 25) are separated after their positioning via miniaturised deflecting electrodes (14, 25, 34) or deflected from their swimming direction after release from the stably held position.

7. Process according to one of the previous process claims, **characterised in that** in the region, in which the driving force of the monocellular organism movements is just compensated, detectors (44) or optical windows are provided, with which the parameters characterising the monocellular organisms, in particular quantification of their swimming movement, are recorded.

8. Process according to one of the previous process claims, **characterised in that** some of the evaluated monocellular organisms are separated and transferred to a separated-off microcompartment.

9. Process according to one of the previous process claims, **characterised in that** the braking electrodes (13, 23, 33, 43) and deflecting electrodes (14, 24, 34) are controlled or regulated via a computer or microprocessor.

10. Process according to one of the previous process claims, **characterised in that** the electric alternating fields or migrating fields are produced at the electrodes (11a, 11b; 13) via signals of a frequency greater than 0.1 MHz with amplitudes between 0.1 V and 40 V.

11. Process according to one of the previous process claims, **characterised in that** bacteria or sperm of the widest variety of animal types or human sperm are used as moveable monocellular organisms.

12. Process according to one of the previous process claims, **characterised in that** in addition to the electric-hydrodynamic holding of the movable monocellular organisms, chemical or optical signals are emitted in order to cause a change in direction for the monocellular organisms.

## Revendications

1. Procédé pour le positionnement stabilisé ou la séparation de corps unicellulaires activement mobiles dans des milieux fluides, dans lequel les corps unicellulaires (15, 25, 45)
(a) sont alignés et freinés par l'intermédiaire d'une ou plusieurs électrodes (11, 21, 31, 41) allongées de l'ordre du micromètre, au moyen de champs à haute fréquence variables ou à ondes progressives ;
(b) sont retardés dans leur mouvement en avant jusqu'à être totalement arrêtés avec une ou plusieurs électrodes de freinage (13, 23, 33, 43) - dont les dimensions typiques correspondent aux dimensions des électrodes d'alignement (11, 21, 31, 41) - pour pouvoir dans cet état de positionnement enregistrer et évaluer un ou des mouvement(s) d'organelles ou pour pouvoir prélever sélectivement des corps unicellulaires individuels au moyen de microéléments à introduire.

2. Procédé selon la revendication 1, **caractérisé en ce qu'**un flux (12, 22, 32) en sens inverse des corps unicellulaires (15, 25, 45) est produit de l'extérieur ou par l'intermédiaire d'une commande électrique des microélectrodes (11, 21, 31, 41).

3. Procédé selon l'une des revendications précédentes, **caractérisé en ce que** deux ou plusieurs corps unicellulaires sont mis en position ou formés en groupes simultanément.

4. Procédé selon l'une des revendications précédentes, dans lequel les corps unicellulaires individuels sont prélevés sélectivement au moyen de tubes capillaires.

5. Procédé selon l'une des revendications précédentes, dans lequel le mouvement d'organelles est le mouvement de poils, de cils ou de flagelles (15a).

6. Procédé selon l'une des revendications précédentes, **caractérisé en ce que,** par l'intermédiaire d'électrodes de déviation miniaturisées (14, 25, 34), les corps unicellulaires (15, 24, 45) sont séparés après leur positionnement ou sont déviés de leur direction de nage après être sortis de leur position stabilisée.

7. Procédé selon l'une des revendications précédentes, **caractérisé en ce que**, dans la zone dans laquelle la force entraînante du mouvement des corps unicellulaires est exactement compensée, il est prévu des détecteurs (44) ou des fenêtres optiques avec lesquels les paramètres caractérisant les corps unicellulaires, notamment la quantification de leur mouvement de nage, sont détectés.

8. Procédé selon l'une des revendications précédentes, **caractérisé en ce que** quelques-uns des corps unicellulaires évalués sont séparés et transférés dans un microcompartiment distinct.

9. Procédé selon l'une des revendications précédentes, **caractérisé en ce que** les électrodes de freinage (13, 23, 33, 43) et les électrodes de déviation (14, 24, 34) sont contrôlées ou commandées par l'intermédiaire d'un ordinateur ou d'un microprocesseur.

10. Procédé selon l'une des revendications précédentes, **caractérisé en ce que** les champs électriques variables ou progressifs sont produits au niveau des électrodes (11a, 11b ; 13) par l'intermédiaire de signaux d'une fréquence supérieure à 0,1 MHz avec des amplitudes comprises entre 0,1 V et 40 V.

11. Procédé selon l'une des revendications précédentes, **caractérisé en ce qu'**on utilise comme corps unicellulaires mobiles des bactéries ou des spermatozoïdes de différentes espèces animales ou des spermatozoïdes humains.

12. Procédé selon l'une des revendications précédentes, **caractérisé en ce que**, en plus de l'immobilisation électro-hydrodynamique des corps unicellulaires mobiles, des signaux chimiques ou optiques sont émis pour provoquer un changement de direction des corps unicellulaires.
